Europäisches Patentamt

European Patent Office

Office européen des brevets

(19)

(11) Veröffentlichungsnummer: **0 104 191**
**B1**

(12) **EUROPÄISCHE PATENTSCHRIFT**

(45) Veröffentlichungstag der Patentschrift:
**06.11.85**

(21) Anmeldenummer: **83900925.5**

(22) Anmeldetag: **23.03.83**

(86) Internationale Anmeldenummer:
**PCT/EP 83/00085**

(87) Internationale Veröffentlichungsnummer:
**WO 83/03355 (13.10.83 Gazette 83/24)**

(51) Int. Cl.⁴: **A 61 M 25/00**

(54) **VORRICHTUNG ZUR DRAINAGE DER HARNBLASE.**

(30) Priorität: **29.03.82 DE 3211576**

(43) Veröffentlichungstag der Anmeldung:
**04.04.84 Patentblatt 84/14**

(45) Bekanntmachung des Hinweises auf die Patenterteilung:
**06.11.85 Patentblatt 85/45**

(84) Benannte Vertragsstaaten:
**FR GB**

(56) Entgegenhaltungen:
**DE - A - 1 491 624**
**FR - A - 2 023 057**
**US - A - 3 640 281**
**US - A - 3 713 447**
**US - A - 3 920 023**

(73) Patentinhaber: **BENDER, Hans Georg, Stüttgener Strasse 80, D-4040 Neuss (DE)**

(72) Erfinder: **BENDER, Hans Georg, Stüttgener Strasse 80, D-4040 Neuss (DE)**

(74) Vertreter: **Patentanwaltsbüro Cohausz & Florack, Schumannstrasse 97, D-4000 Düsseldorf 1 (DE)**

## Beschreibung

Die Erfindung betrifft eine Vorrichtung zur Drainage der Harnblase durch die Bauchdecke mit einem stabilen, gekrümmten Rohr, durch das ein Katheter in die Harnblase einbringbar ist, wobei der Katheter am vorderen Ende eine Spitze, durch die Harnblasenwand und Bauchdecke durchstossbar sind, trägt und mit dieser Spitze im Rohr geschützt einliegt.

Es ist bekannt, einen Katheter durch die Harnröhre in die Harnblase einzuführen, um diese zu entleeren. Da ein solcher Katheter meist längere Zeit dort verbleiben muss, besteht eine erhöhte Gefahr von Infektionen der Harnblase, der Harnwege und der Nieren. Auch führt ein längeres Verweilen des Katheters in der Harnröhre später nach Entfernung zu Störungen der Entleerung der Harnblase.

Um die Nachteile eines in der Harnröhre liegenden Katheters (transurethrale Ableitung) zu meiden, kann man einen Katheter durch die Bauchdecke zur Harnblase legen (suprapubische Ableitung). Eine Vorrichtung hierfür ist aus der DE-A Nr. 1954956 bekannt. Der dort beschriebene Katheter wird von aussen durch die Bauchdecke und die Blasenwand gestossen und auf demselben Weg wieder entfernt. Um vor dem Einführen des Katheters die Lage der Harnblase zu ermitteln, ist es erforderlich, die Harnblase durch die Harnröhre hindurch aufzufüllen. Aber auch hierdurch ist eine absolut sichere Beurteilung der Lage der Harnblase von aussen nicht möglich, so dass beim Durchstossen eine Verletzungsgefahr von Nachbarorganen besteht. Auch können von aussen nach innen Keime eingebracht werden.

Aus der US-A Nr. 3920023 ist eine Vorrichtung zur Drainage der Harnblase der eingangs genannten Art bekannt, bei der der Katheter am vorderen Ende eine Spitze trägt und mit seiner Spitze in einem Rohr einliegt. Im hinteren Bereich liegt der Katheter in einem weiteren Rohr, das ein Griffteil aufweist, so dass ein Einschieben des zweiten Rohres in das erste den Katheter nach vorne bringt und die Spitze aus dem vorderen Ende des ersten Rohres austreten lässt. Die Übertragung des Druckes vom zweiten Rohr zur Spitze erfolgt über den Katheter, der hierzu ungeeignet ist, da er flexibel sein muss. Ferner ist der Katheter vor Keimen nicht geschützt, und das vordere Ende des ersten Rohres kann Verletzungen in der Harnröhre verursachen.

Aufgabe der Erfindung ist es, eine einfach und sicher handhabbare Vorrichtung zum Einbringen eines Katheters zum Entleeren der Harnblase zu schaffen, die die Gefahr von Infektionen und Störungen der Blasenentleerung verringert und eine Verletzungsgefahr von Harnröhre, Harnblase und Nachbarorganen ausschliesst.

Diese Aufgabe wird erfindungsgemäss dadurch gelöst, dass auch das stabile Rohr aus seinem vorderen Ende die Spitze mitträgt und dass die Spitze von einer ein stumpfes Ende bildenden Hülle umgeben ist, die durch ein am hinteren Rohrende betätigbares Zugmittel von der Spitze entfernbar ist. Die erfindungsgemässe Vorrichtung ermöglicht eine exakte Positionsfindung der Blase und der Durchtrittsstelle des Katheters von der Blase nach aussen durch die Bauchdecke, indem das noch durch die Hülle geschützte stumpfe freie Ende der Vorrichtung von innen gegen die Bauchdecke gedrückt wird und damit von aussen sichtbar wird, wo Harnblase und Bauchdecke durchstossen werden können. Dies bedeutet eine sichere Lagebeurteilung der Harnblase und damit keine Verletzungsgefahr für Nachbarorgane. Die Vorrichtung verbleibt nur kurze Zeit in der Harnröhre, so dass eine Infektionsgefahr und Entleerungsstörungen der Harnblase auch dann gering sind, wenn der Katheter längere Zeit in der Harnblase verbleibt. Auch kann bei einer von der Scheide aus vorgenommenen Operation das Einbringen des Katheters vom Operationsfeld her durchgeführt werden. Statt mehrerer Instrumente ist nur noch ein einziges in Form der erfindungsgemässen Vorrichtung erforderlich.

Der Katheter kann von hoher Flexibilität sein und liegt geschützt vor Keimen in dem Rohr ein, das allseits geschlossen sein kann. Das vordere Ende der Vorrichtung bildet eine ideale Abrundung, die sicher Verletzungen der Harnröhre während des Durchschiebens ausschliesst.

Aus der US-A Nr. 3640281 ist es an sich bekannt, das vordere Ende einer Drainagevorrichtung mit einem einen Katheter enthaltenen Rohr durch eine halbkugelförmige Kappe zu verschliessen. Aber hier werden Bauchdecke und Blasenwand von aussen nach innen durch ein Skalpell durchstossen. Dies erfordert eine erhöhte Geschicklichkeit, und ferner können Keime von aussen eingebracht werden.

Ein sehr sicheres Entfernen der Abdeckung der Spitze wird dadurch erreicht, dass das Zugmittel eine das Rohr umgebende Verlängerung der Hülle ist.

Der Katheter kann erst nach Durchstossen der Bauchdecke in die Vorrichtung eingebracht werden. Besonders vorteilhaft ist es aber, wenn der Katheter über seine gesamte Länge im Inneren des Rohres einliegt. Dies bedeutet einen geringeren Arbeitsaufwand, eine bessere Hygiene und keine zusätzlichen Arbeitsmittel.

Zum Abdecken der Spitze wird vorgeschlagen, dass die Hülle einen die Spitze umgebenden ringförmigen Bereich aufweist, der durch die Spitze erweiterbar ist. Alternativ kann auch die Hülle einen vorderen Wandbereich aufweisen, der von der Spitze durchstossbar ist.

Vorzugsweise wird vorgeschlagen, dass an der Rückseite der Spitze der Katheter lösbar befestigt ist. Hierdurch wird auch bei Abziehen des Rohres vom Katheter die sichere Lage des Katheters beibehalten.

Zur Sicherung der Lage des inneren Katheterendes kann der Katheter am in der Harnblase verbleibenden Ende einen entfaltbaren Ballon aufweisen. Dabei kann das Innere des Ballons mit einer zweiten Leitung im Katheter verbunden sein. Ein Einbringen eines Druckmittels von aussen in den Ballon erübrigt sich, wenn im Ballon und/oder in der zweiten Leitung ein Überdrück herrscht, durch den der Ballon entfaltbar ist.

Auch wird vorgeschlagen, dass an dem der Spitze gegenüberliegenden Ende das Rohr eine Öffnung aufweist. Durch diese Öffnung kann über das Rohrinnere eine Flüssigkeit in die Blase eingeführt werden, um die Blase zu füllen und damit die Blase näher an die Bauchdecke zu bringen und die Lage der Blase leichter bestimmen zu können. Bei dieser Ausführung muss das Rohr nahe der Spitze eine Austrittsöffnung aufweisen, die durch Hülle und Zugmittel nicht geschlossen ist.

Ein leichtes Entfernen der Hülle von der Spitze wird dadurch erreicht, dass an dem der Spitze gegenüberliegenden Ende Rohr und Zugmittel je ein Griffteil aufweisen und die Hülle zusammen mit dem Zugmittel in der Längserstreckung kürzer ausgeführt sind als das Rohr.

Zwei Ausführungsbeispiele der Erfindung sind in den Zeichnungen in vergrössertem Massstab dargestellt. Es zeigen:

Fig. 1 einen Längsschnitt durch ein erstes Ausführungsbeispiel der Vorrichtung und

Fig. 2 einen Längsschnitt durch eine zweites Ausführungsbeispiel in einem Ausschnitt.

Ein gebogenes Rohr 1 von 3-6 mm Aussendurchmesser aus steifem Kunststoff oder Metall trägt am vorderen Ende eine Spitze 2, die mit einem an der Rückseite angeordneten Gewinde 3 in ein Innengewinde des Rohres eingeschraubt ist. An der Rückseite der Spitze springt ein Zapfen 4 vor, der in den Hauptlängskanal 5 eines Katheters 6 gesteckt ist, der aus Gummi, insbesondere Latex besteht. Der Katheter 6 liegt über seine gesamte Länge im Rohr 1 ein und weist an der der Spitze 2 gegenüberliegenden Seite zum Kanal 5 führende Eintrittsöffnungen 7 und einen Ballon 8 auf, der mit einem zweiten Längskanal 9 des Katheters 6 verbunden ist.

An dem der Spitze abgewandten Ende des Katheters 6 befindet sich ein Flansch 10 oder radiale Griffteile und ferner eine Öffnung 11, in die eine Flüssigkeit eindrückbar ist, die durch das Innere des Rohres 1 fliesst und nahe der Spitze 2 durch eine Öffnung 12 des Rohres 1 und durch eine Öffnung 13 einer Hülle 14 austreten kann. Die Hülle 14 umgibt als Rohr oder Schlauch geringerer Wandstärke als das Rohr 1 das gesamte Rohr bis auf einen dem Griff 10 nahe liegenden Bereich 19. Am hinteren Ende der äusseren Schutzhülle 14 ist ein Flansch oder radialer Griff 15 angeformt. Die Schutzhülle 14 umgibt am vorderen Ende mit einer Wölbung die Spitze 2 und ist in diesem Bereich 14b derart dünnwandig und/oder vom Material her weich ausgeführt, dass sie von der Spitze durchstossen werden kann. Hierzu genügt es, die Schutzhülle durch den Griff 15 zum Griff 10 hin zu ziehen.

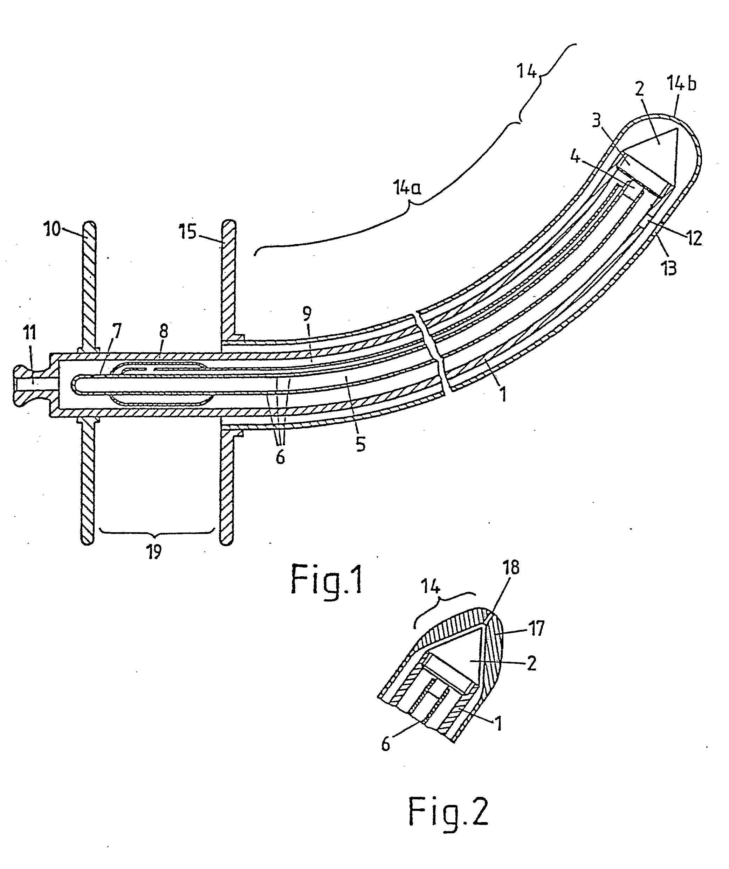

In einem nicht gezeigten Ausführungsbeispiel kann die Hülle 14 allein im vorderen Bereich das Rohr 1 überdecken und durch ein Zugmittel mit dem Griff 15 verbunden sein. Aber auch bei dem in Fig. 1 dargestellten Ausführungsbeispiel ist der ausserhalb des vorderen Endes liegende Bereich 14a der Schutzhülle 14 als ein Zugmittel anzusehen, das die vorne angeordnete Hülle mit dem Griff 15 verbindet.

In dem in Fig. 2 dargestellten Ausführungsbeispiel bildet die Hülle 14 einen die Spitze umgebenden ringförmigen Bereich 17 mit einer zentralen Öffnung 18, die durch die Elastizität des Ringes 17 so lange geschlossen bleibt, bis die Hülle 14 zum hinteren Ende gezogen wird und damit die Spitze 2 die Öffnung 18 aufweitet und der Ring über die Spitze nach hinten gleitet.

Die gesamte Vorrichtung wird mit dem vorderen Ende voran in die Harnröhre so weit eingeschoben, bis das vordere Ende weit in der Harnblase einliegt. Durch die Öffnung 11 wird Flüssigkeit in die Harnblase eingedrückt, bis diese so weit gefüllt ist, dass sie über einen erheblichen Bereich an der Bauchdecke innen anliegt. Darauf wird die mit ihrem hinteren Ende immer noch aussen aus dem Körper ragende Vorrichtung mit dem vorderen stumpfen Ende von innen gegen die Harnblasenwand und die Bauchdecke gedrückt, so dass von aussen diese Stelle sichtbar ist. Hierdurch kann exakt bestimmt werden, dass zwischen Harnblasenwand und Bauchdecke keine weiteren Organe liegen. Ferner kann die genaue Durchstossstelle gewählt werden. Darauf wird der Griff 15 zum Griff 10 gedrückt, so dass die Spitze 2 durch die Schutzhülle 14 nach vorne vordringt, wonach mit der Spitze Harnblasenwand und Bauchdecke von innen nach aussen durchstossen werden. Die Spitze 2 wird nunmehr vom Rohr 1 abgeschraubt, und durch die Spitze wird der Katheter 6 einige Zentimeter herausgezogen. Danach wird der Katheter festgehalten, und Schutzhülle 14 und Rohr 1 werden nach hinten aus der Harnröhre herausgezogen, so dass das hintere Ende des Katheters 6 in der Harnblase und das vordere Ende aussen liegt.

Der Ballon kann nun aufgepumpt und der Katheterkanal 5 an einen Beutel angeschlossen werden. Der Ballon 8 kann auch dadurch zum Entfalten gebracht werden, dass im Kanal 9 ein Überdruck besteht, der erst nach Herausziehen des Katheters 6 aus dem Rohr 1 zur Wirkung kommt.

## Patentansprüche

1. Vorrichtung zur Drainage der Harnblase durch die Bauchdecke mit einem stabilen, gekrümmten Rohr (1), durch das ein Katheter (6) in die Harnblase einbringbar ist, wobei der Katheter am vorderen Ende eine Spitze (2), durch die Harnblasenwand und Bauchdecke durchstossbar sind, trägt und mit der Spitze im Rohr geschützt einliegt, dadurch gekennzeichnet, dass auch das stabile Rohr (1) an seinem vorderen Ende die Spitze (2) mitträgt und dass die Spitze von einer ein stumpfes Ende bildenden Hülle (14) umgeben ist, die durch ein am hinteren Rohrende betätigbares Zugmittel (14a) von der Spitze entfernbar ist.

2. Vorrichtung nach Anspruch 1, dadurch gekennzeichnet, dass das Zugmittel (14a) eine das Rohr (1) umgebende Verlängerung der Hülle (14) ist.

3. Vorrichtung nach einem der Ansprüche 1 oder 2, dadurch gekennzeichnet, dass die Hülle (14) einen die Spitze (2) umgebenden ringförmigen Bereich (17) aufweist, der durch die Spitze erweiterbar ist.

4. Vorrichtung nach einem der Ansprüche 1 bis 3, dadurch gekennzeichnet, dass die Hülle (14) einen vorderen Wandbereich (14b) aufweist, der von der Spitze (2) durchstossbar ist.

5. Vorrichtung nach einem der Ansprüche 1 bis 4, dadurch gekennzeichnet, dass das eine Spitze (2) tragende Rohr (1) einteilig vom vorderen Ende bis zu einem Griffteil (10) durchläuft.

6. Vorrichtung nach einem der Ansprüche 1 bis 5, dadurch gekennzeichnet, dass der Katheter (6) über seine gesamte Länge im Inneren des Rohres (1) einliegt.

7. Vorrichtung nach Anspruch 6, dadurch gekennzeichnet, dass an der Rückseite der Spitze (2) der Katheter (6) lösbar befestigt ist.

8. Vorrichtung nach einem der Ansprüche 1 bis 7, dadurch gekennzeichnet, dass der Katheter (6) am in der Harnblase verbleibenden Ende einen entfaltbaren Ballon (8) aufweist.

9. Vorrichtung nach Anspruch 8, dadurch gekennzeichnet, dass das Innere des Ballons (8) mit einer zweiten Leitung (19) im Katheter (6) verbunden ist.

10. Vorrichtung nach Anspruch 9, dadurch gekennzeichnet, dass im Ballon (8) und/oder in der zweiten Leitung (9) ein Überdruck herrscht, durch den der Ballon entfaltbar ist.

11. Vorrichtung nach einem der Ansprüche 1 bis 10, dadurch gekennzeichnet, dass an dem der Spitze (2) gegenüberliegenden Ende das Rohr (1) eine Öffnung (11) aufweist.

12. Vorrichtung nach einem der Ansprüche 1 bis 11, dadurch gekennzeichnet, dass an dem der Spitze gegenüberliegenden Ende Rohr (1) und Zugmittel (14a) je ein Griffteil (10, 15) aufweisen und die Hülle (14) zusammen mit dem Zugmittel in der Längserstreckung kürzer ausgeführt sind als das Rohr.

## Revendications

1. Dispositif pour le drainage de la vessie au moyen d'un tube (1) courbé et stable à travers lequel un cathéter (6) peut être introduit dans la vessie, le cathéter portant à son extrémité extérieure une aiguille (2) par laquelle peuvent être traversées la paroi de la vessie et la paroi de l'abdomen et étant logé dans le tube avec l'aiguille en étant protégé, caractérisé par le fait que le tube stable (1) également porte l'aiguille (2) à son extrémité antérieure et que l'aiguille (2) est entourée par une gaine (14) formant une extrémité tronquée qui peut être écartée de l'aiguille par un moyen d'entraînement (14a) pouvant être actionné à l'extrémité arrière du tube.

2. Dispositif selon la revendication 1, caractérisé par le fait que le moyen d'entraînement (14a) est un prolongement de la gaine (14) entourant le tube (1).

3. Dispositif selon la revendication 1 ou 2, caractérisé par le fait que la gaine (14) présente une zone (17) annulaire entourant l'aiguille (2) qui peut être écartée par l'aiguille.

4. Dispositif selon l'une des revendications 1 à 3, caractérisé par le fait que la gaine (14) présente une zone de paroi (14b) antérieure qui peut être traversée par l'aiguille (2).

5. Dispositif selon l'une des revendications 1 à 4, caractérisé par le fait que le tube (1) portant une aiguille (2) s'étend en une seule pièce de l'extrémité antérieure à une partie de poignée (10).

6. Dispositif selon l'une des revendications 1 à 5, caractérisé par le fait que le cathéter (6) est logé sur toute sa longueur à l'intérieur du tube (1).

7. Dispositif selon la revendication 6, caractérisé par le fait que le cathéter (6) est fixé de manière démontable à la face arrière de l'aiguille (2).

8. Dispositif selon l'une des revendications 1 à 7, caractérisé par le fait que le cathéter (6) présente, à l'extrémité restant dans la vessie, un ballon déployable (8).

9. Dispositif selon la revendication 8, caractérisé par le fait que l'intérieur du ballon (8) est relié à un second conduit (19) dans le cathéter (6).

10. Dispositif selon la revendication 9, caractérisé par le fait qu'une surpression règne dans le ballon (8) et/ou dans le second conduit (9) grâce à laquelle le ballon peut être déployé.

11. Dispositif selon l'une des revendications 1 à 10, caractérisé par le fait que le tube (1) présente, à l'extrémité opposée à l'aiguille (2), une ouverture (11).

12. Dispositif selon l'une des revendications 1 à 11, caractérisé par le fait que, à l'extrémité opposée à l'aiguille, le tube (1) et le moyen d'entraînement (14a) présentent chacun une partie de poignée (10, 15) et la gaine (14) ainsi que le moyen d'entraînement sont réalisés plus courts que le tube en étendue longitudinale.

## Claims

1. Device for draining the bladder through the roof of the stomach with a strong, curved tube (1) through which a catheter (6) can be fed into the bladder, whereby the lower end of the catheter has a point (2) which lies protected in the tube and which can be pushed through the wall of the bladder and the wall of the stomach, characterized by the fact that the strong tube (1) also has a point (2) at its front end and that the point is surrounded by a sleeve (14) which forms a blunt end and which can be removed from the pointed end by a pulling device (14a) operated from the other end of the tube.

2. Device according to Claim 1, characterized by the fact that the pulling device (14a) is an extension of the sleeve (14) which surrounds the tube (1).

3. Device according to Claim 1 or 2, characterized by the fact that the sleeve (14) has a circular area (17) which surrounds the pointed tip (2) and which can be extended by the pointed tip.

4. Device according to one of Claims 1 to 3, characterized by the fact that the sleeve (14) has a front wall area (14b) which can be penetrated by the pointed tip (2).

5. Device according to one of Claims 1 to 4, characterized by the fact that the tube (1) with the pointed tip (2) is of one-part construction from the front end to a handle section (10).

6. Device according to one of Claims 1 to 5, characterized by the fact that the entire length of the catheter (6) lies inside the tube (1).

7. Device according to Claim 6, characterized by the fact that the catheter (6) is connected to the rear of the pointed tip (2) so that it can be released.

8. Device according to one of Claims 1 to 7, characterized by the fact that the catheter (6) has a folded balloon (8) at the end which remains in the bladder.

9. Device according to Claim 8, characterized by the fact that the inside of the balloon (8) is connected to a second tube (19) in the catheter (6).

10. Device according to Claim 9, characterized by the fact that an overpressure exists in the balloon (8) and/or in the second tube (9) through which the balloon can be inflated.

11. Device according to one of Claims 1 to 10, characterized by the fact that there is an opening (11) at the end of the tube (1) opposite to the pointed tip (2).

12. Device according to one of Claims 1 to 11, characterized by the fact that the end of the tube (1) opposite to the pointed tip and the pulling device (14a) each have a handle (10, 15) and the sleeve (14) together with the pulling device are shorter in length than the tube.

Fig.1

Fig.2